# EUROPÄISCHE PATENTANMELDUNG

(11) **EP 0 621 045 A2**
(43) Veröffentlichungstag der Anmeldung: **26.10.1994**
(21) Anmeldenummer: 94105454.6
(22) Anmeldetag: 08.04.1994
(51) Int. Cl.: A61L 29/00

(54) **Katheter und Schläuche für medizinische Anwendungen**

(30) Priorität: 21.04.1993 DE 4312893
(71) Anmelder: BAYER AG, 51368 Leverkusen (DE)
(72) Erfinder: Pudleiner, Heinz Dr., D-47800 Krefeld (DE); Köhler, Burkhard Dr., D-47829 Gladbach 2 (DE); Dhein, Rolf Dr., D-47800 Krefeld (DE); Hugl, Herbert, Dr., D-51467 Bergisch Gladbach 2 (DE)

(57) **Zusammenfassung**

Neue Katheter und Schläuche für medizinische Anwendungen können aus Copolymeren von Olefinen und ungesättigten Carbonsäuren, deren Schmelzbereich, gemessen durch DSC, bei Temperaturen unterhalb von 32°C beginnt, hergestellt werden. Diese Copolymere haben einen Ethylengehalt von 60 bis 94,5 Gew.-%, einen Gehalt von 5 bis 39,5 Gew,-% an Comonomeren aus der Gruppe von C₃-C₂₂-α-Olefinen, C₄-C₆-Vinylestern und C₄-C₈-(Meth)Acrylsäureestern und einen Gehalt an ungesättigten Carbonsäuren von 0,5 bis 10 Gew.-%, wobei sich die Prozent-Angaben auf das Gesamtgewicht des Copolymer beziehen. Der Anteil der Carboxylgruppen aus den ungesättigten Säuren ist zu 10 bis 100 % durch anorganische Kationen neutralisiert.

## Beschreibung

Die vorliegende Erfindung bezieht sich auf Katheter und Schläuche für medizinische Anwendungen, die aus Copolymeren von Olefinen und ungesättigten Carbonsäuren, deren Schmelzbereich, gemessen durch DSC, bei Temperaturen von unterhalb 32°C beginnt, hergestellt werden. Die Erfindung betrifft demnach weiterhin die Verwendung solcher Copolymerer zur Herstellung von Kathetern und Schläuchen für medizinische Anwendungen.

In WO 86/03980 ist die Herstellung von Kathetern aus Blends zweier Polymerer beschrieben. Das erste Polymer hat eine Glastemperatur von oberhalb 62°C; durch Zumischung des zweiten Polymer, welches ein Blockcopolymer ist, wird die Glastemperatur des Blends zwischen 20 und 62°C eingestellt. Solche Katheter zeichnen sich durch eine Erweichung bei Körpertemperatur aus, wodurch die Gefahr der Verletzung bei der medizinischen Anwendung solcher Katheter verringert wird.

Die Herstellung des Polymerblends gemäß WO 86/03980 ist ein zusätzlicher Verarbeitungsschritt. Es war wünschenswert, Katheter und andere Schläuche für medinizinische Anwendungen, beispielsweise Blutschläuche, ohne den vorgelagerten Produktionsschritt der Herstellung des Polymerblends aus nur einem Polymer herstellen zu können; solche Materialien sollten eine Erweichung bei Temperaturen um 37°C, also der Körpertemperatur des Menschen, aufweisen. Als Erweichung wird bei Polymeren der Beginn des größeren Schmelzbereichs angesehen.

Aus M. Costello, B. Stanzweski, P. Vriesman , T. Lucas, S. Srinivasan und P.N. Sawyer "Correlations between electrochemical and antithrombogenic characteristics of polyelectrolyte materials" in Trans. Amer. Soc. Artif. Int. Organs, 1970, s. 1 bis 6 ist bekannt, daß Ethylen/Acrylsäure-Copoylmere und deren Neutralisations- oder Teilneutralisationsprodukte antithrombogene Eigenschaften haben können. Für Anwendungen wie z.B. Gefäßprothesen, bei denen die Steifigkeit eine gewünschte Materialeigenschaft ist, können solche Polymere geeignet sein. Wie das Vergleichsbeispiel weiter unten zeigt, sind jedoch einfache Copolymere, so wie sie auch oben beschrieben werden, für Katheteranwendungen ungeeignet, da solche Polymere keine Vorerweichungseigenschaften haben und die Katheterspitze von aus solchen Materialien hergestellten Kathetern die Blutgefäßinnenwand verletzen kann.

Die Erfindung betrifft Katheter und Schläuche für medizinische Anwendungen aus Copolymeren von olefinischen Monomeren und ungesättigten Carbonsäuren, deren Schmelzbereich, gemessen durch DSC, bei Temperaturen von unter 32°C beginnt, mit einem Ethylengehalt von 60 bis 94,5 Gew.-%, einem Gehalt von 5 bis 39,5 Gew.-% an Comonomeren aus der Gruppe von C₃-C₂₂-α-Olefinen, C₄-C₆-Vinylestern und C₄-C₈-(Meth)Acrylsäureestern und einem Gehalt an ungesättigten Carbonsäuren von 0,5 bis 10 Gew.-%, wobei sich die Prozent-Angaben auf das Gesamtgewicht des Copolymeren beziehen und wobei der Anteil der Carboxylgruppen aus den ungesättigten Säuren zu 10 bis 100 % durch anorganische Kationen neutralisiert ist.

Als olefinisches Monomer kommt in erster Linie Ethylen mit einem Anteil von 60 bis 94,5 Gew.-%, bevorzugt 80-89,5 Gew.-%, bezogen auf das Gesamtgewicht des Copolymer, in Frage.

Weitere olefinische Monomere sind eines oder mehrere aus der Gruppe von C₃-C₂₂-α-Olefinen, C₄-C₆-Vinylestern und C₄-C₈-(Meth)Acrylsäureestern. In bevorzugter Weise liegen diese Comonomeren in einer Menge von 10-19,5 Gew.-% vor. α-Olefine der genannten Art sind beispielsweise Propen, Buten-1, Penten-1, Hexen-1, Octen-1, Decen-1, Dodecen-1, Hexadecen-1 und Eicosen-1. Bevorzugte α-Olefine haben 3 bis 6 C-Atome; besonders bevorzugt unter diesen ist das Propylen.

C₄-C₆-Vinylester sind beispielsweise Vinylacetat, Vinylpropionat, Vinylbutyrat, bevorzugt Vinylacetat.

C₄-C₈-(Meth)Acrylsäureester sind beispielsweise Methylacrylat, Methylmethacrylat, Ethylacrylat, Ethylmethacrylat und Propylacrylat, in bevorzugter Weise Methylacrylat oder Methylmethacrylat.

Von den genannten Comonomeren können eines oder mehrere der genannten Art im Copolymer für die Katheter und medizinischen Schläuche vorliegen. In bevorzugter Weise liegt jedoch Propylen alleine als Comonomer vor.

Ungesättigte Carbonsäuren für die erfindungsgemäß einzusetzenden Copolymere sind Acrylsäure, Methacrylsäure, Crotonsäure, Maleinsäure, Fumarsäure und deren Anhydride, beispielsweise Maleinsäureanhydrid. In bevorzugter Weise liegen Methacrylsäure oder Acrylsäure oder ein Gemisch beider als ungesättigte Carbonsäure im Copolymer vor. Die Carboxylgruppen aus den ungesättigten Säuren bzw. aus deren Anhydriden sind im einzusetzenden Copolymer zu 10 bis 100 %, bevorzugt zu 50-100 % durch anorganische Kationen neutralisiert. Anorganische Kationen hierfür sind beispielsweise solche aus Li, Na, K, Rb, Cs, Mg, Ca, Al, Zn. Es kann sich also um einwertige, zweiwertige oder dreiwertige Kationen handeln. In bevorzugter Weise werden als Kationen solche von Na oder Zn, bevorzugt von Na eingesetzt.

Der Schmelzbereich der Copolymeren und dem entsprechend der erfindungsgemäßen Katheter und Schläuche für medizinische Anwendungen beginnt bei Temperaturen von unterhalb 32°C und liegt insgesamt beispielsweise in einem Bereich von 10 bis 85°C, bevorzugt von 15 bis 80°C. Der Schmelzbereich, insbesondere sein Beginn, wird durch DSC (Differental Scanning Calorimetry) gemessen.

Die Erfindung betrifft gleichermaßen die Verwendung von Copolymeren der oben beschriebenen Art zur Herstellung von Kathetern und Schläuchen für medizinische Anwendungen. Copolymere der genannten Art sind marktgängig und können beispielsweise unter dem Handelsnamen Surlyn® der Fa. DuPont beschafft werden.

### Beispiel 1

Die Herstellung des Katheters erfolgte aus Surlyn 8320® (Fa, DuPont), dessen Schmelzbereich im DSC bei 17°C beginnt, mit einem Schmelzpunkt (Maximum des Schmelzpeaks in DSC) von 63°C.

Die Herstellung der Katheter erfolgte auf einem Einwellenextruder der Firma Stork (⌀ 25 mm) mit einem Kompressionsverhältnis 3:1.

Bei 150°C Massetemperatur konnte ein glatter, klebfreier und transparenter Katheterschlauch mit 1 mm Innen- und 1,7 mm Außendurchmesser erhalten werden,

### Vergleichsbeispiel (nicht erfindungsgemäß)

Aus dem nicht erfindungsgemäßen thermoplastischen Ionomer Surlyn 1802® (Fa. DuPont) wurden unter gleichen Bedingungen wie in Beispiel 1 Katheterschläuche hergestellt.

Bei Surlyn 1802® (Fa. DuPont) (Ethylen/Acrylsäure-Ionomer) setzt die Vorerweichung erst deutlich oberhalb der menschlichen Körpertemperatur ein, wie die Schubmodulkurve zeigt.

Die Katheterschläuche waren vergleichsweise steif und inflexibel.

### Beispiel 2

Die Herstellung des Katheters erfolgte aus Surlyn 8120® (Fa. DuPont), wie im Beispiel 1 beschrieben. Es konnte ein glatter, klebfreier und transparenter Katheterschlauch mit 1 mm Innen- und 1,7 mm Außendurchmesser erhalten werden.

## Patentansprüche

1. Katheter und Schläuche für medizinische Anwendungen aus Copolymeren von olefinischen Monomeren und ungesättigten Carbonsäuren, deren Schmelzbereich, gemessen durch DSC, bei Temperaturen von unter 32°C beginnt, mit einem Ethylengehalt von 60 bis 94,5 Gew.-%, einem Gehalt von 5 bis 39,5 Gew.-% an Comonomeren aus der Gruppe von C₃-C₂₂-α-Olefinen, C₄-C₆-Vinylestern und C₄-C₈-(Meth)Acrylsäureestern und einem Gehalt an ungesättigten Carbonsäuren von 0,5 bis 10 Gew.-%, wobei sich die Prozent-Angaben auf das Gesamtgewicht des Copolymer beziehen und wobei der Anteil der Carboxylgruppen aus den ungesättigten Säuren zu 10 bis 100 % durch anorganische Kationen neutralisiert ist.

2. Katheter und Schläuche nach Anspruch 1, dadurch gekennzeichnet, daß als Comonomer Propylen vorliegt.

3. Katheter und Schläuche nach Anspruch 1, dadurch gekennzeichnet, daß als ungesättigte Carbonsäuren Methacrylsäure oder Acrylsäure oder ein Gemisch beider vorliegt.

4. Katheter und Schläuche nach Anspruch 1, dadurch gekennzeichnet, daß als anorganische Kationen solche von Na oder Zn, bevorzugt von Na vorliegen.

5. Verwendung von Copolymeren aus olefinischen Monomeren und ungesättigten Carbonsäuren, deren Schmelzbereich gemessen durch DSC, bei Temperaturen von unter 32°C beginnt, mit einem Ethylengehalt von 60 bis 94,5 Gew.-%, einem Gehalt von 5 bis 39,5 Gew.-% an Comonomeren aus der Gruppe von C₃-C₂₂-α-Olefinen, C₄-C₆-Vinylestern und C₄-C₈-(Meth)Acrylsäureestern und einem Gehalt an ungesättigten Carbonsäuren von 0,5 bis 10 Gew.-%, wobei sich die Prozent-Angaben auf das Gesamtgewicht des Copolymer beziehen und wobei der Anteil der Carboxylgruppen aus ungesättigten Carbonsäuren zu 10 bis 100 % durch anorganische Kationen neutralisiert ist, zur Herstellung von Kathetern und Schläuchen für medizinische Anwendungen.

6. Verwendung nach Anspruch 5, dadurch gekennzeichnet, daß als Comonomer Propylen vorliegt.

7. Verwendung nach Anspruch 5, dadurch gekennzeichnet, daß als ungesättigte Carbonsäuren Methacrylsäure oder Acrylsäure oder ein Gemisch beider vorliegt.

8. Verwendung nach Anspruch 5, dadurch gekennzeichnet, daß als anorganische Kationen solche von Na oder Zn, bevorzugt von Na vorliegen.
